# EUROPEAN PATENT SPECIFICATION

(11) **EP 2 480 129 B1**
(45) Date of publication and mention of the grant of the patent: **06.11.2019**
(21) Application number: 10819580.1
(22) Date of filing: 25.09.2010
(51) Int. Cl.: A61B 5/00, A61B 8/12, A61B 5/02

(54) **DEVICE FOR DETERMINING THE LIKELIHOOD OF A PATIENT HAVING A CLINICALLY SILENT EVENT BASED ON ASCERTAINED PHYSIOLOGICAL PARAMETERS**
VORRICHTUNG ZUR BESTIMMUNG DER WAHRSCHEINLICHKEIT EINES ASYMPTOMATISCHEN EREIGNISSES AUF BASIS GESICHERTER PHYSIOLOGISCHER PARAMETER
DISPOSITIF POUR DÉTERMINER LE RISQUE D'UN PATIENT DE SUBIR UN ÉVÉNEMENT CLINIQUE SILENCIEUX EN FONCTION DE PARAMÈTRES PHYSIOLOGIQUES CONSTATÉS

(30) Priority: 25.09.2009 US 246043 P
(43) Date of publication of application: 01.08.2012
(73) Proprietor: Volcano Corporation, Rancho Cordova, CA 95670 (US)
(72) Inventor: MARGOLIS, Marja, Coral Gables Florida 33143 (US); VINCE, Geoffrey, Poway California 92064 (US); NAIR, Anuja, Bedford Massachusetts 01730 (US)
(74) Representative: de Haan, Poul Erik
(86) International application number: PCT/US2010/050316
(87) International publication number: WO 2011/038305

(56) References cited:
- WO-A1-2009/009140
- WO-A1-2009/032206
- US-A1- 2003 120 134
- US-A1- 2004 077 950
- US-A1- 2005 043 614
- US-A1- 2006 241 465
- US-A1- 2007 260 141
- US-A1- 2007 260 141
- US-A1- 2008 008 366
- US-A1- 2010 081 941
- US-B1- 6 503 202
- Morteza Naghavi ET AL: "From Vulnerable Plaque to Vulnerable Patient-Part III: Executive Summary of the Screening for Heart Attack Prevention and Education (SHAPE) Task Force Report", AMERICAN JOURNAL OF CARDIOLOGY., vol. 98, no. 2, 1 July 2006 (2006-07-01), pages 2-15, XP055573622, US ISSN: 0002-9149, DOI: 10.1016/j.amjcard.2006.03.002
- Stefan Möhlenkamp ET AL: "On the paradox of exercise: coronary atherosclerosis in an apparently healthy marathon runner", NATURE CLINICAL PRACTICE CARDIOVASCULAR MEDICINE, vol. 4, no. 7, 1 July 2007 (2007-07-01), pages 396-401, XP055573703, GB ISSN: 1743-4297, DOI: 10.1038/ncpcardio0926
- Stefan Möhlenkamp ET AL: "On the paradox of exercise: coronary atherosclerosis in an apparently healthy marathon runner", NATURE CLINICAL PRACTICE CARDIOVASCULAR MEDICINE, vol. 4, no. 7, 1 July 2007 (2007-07-01), pages 396-401, XP55573703, GB ISSN: 1743-4297, DOI: 10.1038/ncpcardio0926
- LANKEREN ET AL: "Plaque area increase and vascular remodeling contribute to lumen area change after percutaneous transluminal angioplasty of the femoropopliteal artery: An intravascular ultrasound study", JOURNAL OF VASCULAR SURG, C.V. MOSBY CO., ST. LOUIS, MO, US, vol. 29, no. 3, 1 March 1999 (1999-03-01), pages 430-441, XP005700455, ISSN: 0741-5214, DOI: 10.1016/S0741-5214(99)70271-5
- Hiroshi Doi ET AL: "Impact of In-Stent Minimal Lumen Area at 9 Months Poststent Implantation on 3-Year Target Lesion Revascularization-Free Survival : A Serial Intravascular Ultrasound Analysis From the TAXUS IV, V, and VI Trials", [Circulation / Cardiovascular interventions] Circulation : journal of the American Heart Association, vol. 1, no. 2, 1 October 2008 (2008-10-01) , pages 111-118, XP055573832, US ISSN: 1941-7640, DOI: 10.1161/CIRCINTERVENTIONS.108.784660
- Hiroshi Doi ET AL: "Impact of In-Stent Minimal Lumen Area at 9 Months Poststent Implantation on 3-Year Target Lesion Revascularization-Free Survival : A Serial Intravascular Ultrasound Analysis From the TAXUS IV, V, and VI Trials", [Circulation / Cardiovascular interventions] Circulation : journal of the American Heart Association, vol. 1, no. 2, 1 October 2008 (2008-10-01) , pages 111-118, XP55573832, US ISSN: 1941-7640, DOI: 10.1161/CIRCINTERVENTIONS.108.784660
- Andreas König ET AL: "Technology Insight: in vivo coronary plaque classification by intravascular ultrasonography radiofrequency analysis", NATURE CLINICAL PRACTICE CARDIOVASCULAR MEDICINE, vol. 5, no. 4, 26 February 2008 (2008-02-26), pages 219-229, XP055573881, GB ISSN: 1743-4297, DOI: 10.1038/ncpcardio1123

## Description

### 1. Field of the invention

This invention relates to improved devices and methods for treating atherosclerosis and more particularly to devices and methods for predicting clinical outcomes for patients based on an evaluation of ascertained physiological parameters related to atherosclerosis.

### 2. Description of Related Art

US 6,503,202 B1 describes medical diagnostic ultrasound methods and systems for automated flow analysis. Multiple cross-sectional areas along a vessel are determined automatically. A processor locates an abnormality as a function of the multiple cross-sectional areas, such as identifying a cross-sectional area that is a threshold amount less than an average cross-sectional area. The abnormal area is highlighted on the display to assist with medical diagnosis.

US 2007/0260141 A1 describes a system and method for automatically classifying plaque lesions. A plaque classification application applies a plaque classification criterion to at least one graphical image, comprising a map of spectrally-analyzed characterized tissue of a vessel cross-section, to render an overall plaque classification for the slice or set of slices, covering a 3D volume. The plaque classification is based upon the amount and location of each characterized tissue type (e.g., necrotic core-NC).

US 2004/0077950 A1 refers to methods and devices for characterizing tissue in vivo, e.g., in walls of blood vessels, to determine whether the tissue is healthy or diseased, and include methods of displaying results with or without thresholds.

WO 2009/009140 A1 describes a method for determination of the risk of atherothrombosis that includes determining blood shear stress based on blood viscosity and comparing the blood shear stress to a critical threshold blood shear stress indicative of the propensity of plaque to rupture.

US 2008/0008366 A1 describes a method of combining data from multi-modality imaging to provide simultaneous processing, visualization and navigation of both functional and anatomical image information, such as, for example, in cardiac studies.

US 2006/0241465 A1 describes system and method for providing a vascular image wherein a single composite display simultaneously provides a first view of a patient including an angiogram image and a second view including an intravascular image rendered from information provided by an imaging probe mounted on a distal end of a flexible elongate member.

US 2010/0081941 A1 refers to methods and apparatus for improving measurements of cardiovascular health status in a given individual. The comprehensive assessment of cardiovascular health includes at least two components: risk factor assessment based on epidemiologic studies and functional status of the individual.

Coronary Artery Disease ("CAD") is the most common type of heart disease and is the leading cause of death in the United States for both men and women. CAD is the manifestation of a condition where plaque builds up inside the coronary arteries (the arteries that supply oxygen-rich blood to the heart). Plaque is made up of fat, cholesterol, calcium, and other substances found in the blood and as a result of inflammation in artery walls. When plaque builds up in the arteries, the condition is called atherosclerosis. The buildup of plaque narrows the arteries and reduces blood flow to the heart muscle. Because plaque narrows the arteries, it also makes it more likely that blood clots will form in the arteries. These blood clots can partially or completely block blood flow.

Also, when coronary arteries are narrowed or blocked, oxygen-rich blood can't reach the heart muscle. This can cause a condition called angina or even result in a heart attack. Angina is chest pain or discomfort that occurs when not enough oxygen-rich blood is flowing to an area of the heart muscle. A heart attack occurs when blood flow to an area of your heart muscle is completely blocked. This prevents oxygen-rich blood from reaching that area of heart muscle and, as a result, the oxygen-starved area of the heart dies. Over time, CAD can weaken the heart muscle and lead to heart failure and arrhythmias. Heart failure is a condition where the heart is weakened and as a result can't pump an adequate amount of blood throughout the body.

As mentioned above, atherosclerosis is the condition where plaque builds up in the arteries. Besides just narrowing the arteries, atherosclerosis is a chronic inflammatory disorder. This disorder involves vascular, metabolic and immune systems and manifests itself in both local and systemic ways. In addition, the plaque itself has considerable variability in its morphology (composition of the plaque). The main plaque components can be characterized as being fibrotic, fibrous with higher lipid or fat content, dense calcium or necrotic core. It is common to have more than one or even all of these plaque components together in a single lesion.

There are several physiological and anatomical parameters related to coronary arteries and the buildup of plaque. Plaque Burden is one of these anatomical parameters. Plaque Burden is defined as: ((vessel area - lumen area) / vessel area) ^{∗} 100 and is expressed as a percentage. The vessel area is the cross-sectional area of the blood vessel not taking into account any buildup of plaque. The lumen area is the cross-sectional area of the blood vessel taking into account the buildup of plaque so that the lumen area is the area that is available for blood flow. For example, if the vessel area is 5 mm² and there is sufficient plaque buildup to produce a lumen area of 3 mm² the resulting Plaque Burden is 40% ((5 mm²⁻ 3 mm²)/ 5 mm²)^{∗}100. A clinically useful limit for the Plaque Burden is about 70%. This "70%" figure is recognized in the art as an amount indicating an intermediate lesion. A Plaque Burden of about 55% is also recognized in the art as the boundary of an insignificant lesion.

Another parameter of interest is the Minimum Lumen Area "MLA". The Minimum Lumen Area is essentially the smallest lumen cross-sectional area in an area of interest along the length of the artery. An area of interest is typically the section of artery that is being imaged. A clinically useful limit for the Minimum Lumen Area is about 4 mm². Minimum Lumen Area equal to or less than about 4 mm² are considered small and hence likely to reduce flow of blood (oxygen) to heart muscle.

A particularly risky combination of plaque elements is known as a thin-cap fibroatheroma ("TCFA"). TCFA is a plaque lesion having a thin coating of fibrous tissue covering a large necrotic core. The thin coating is between the necrotic core and the lumen of the artery. What makes a TCFA troubling is that if the thin fibrous coating (the "thin cap") ruptures, the underlying material of the necrotic core is then free to be discharged into the bloodstream where it can move downstream becoming attached to a narrow section of the artery or to another plaque lesion and result in thrombus or blot clot formation thereby partially or totally occluding the flow of blood through that artery. When there is already a narrowing in the artery, there is a higher likelihood of an undesirable event occurring should the TCFA rupture and discharge necrotic core into the artery.

Further, when a TCFA ruptures there is often a build up of thrombus at the rupture site itself. This buildup of thrombus also narrows the lumen of the artery. This is particularly problematic where there has been a series of TCFA ruptures at a particular site so that there is an accumulation of thrombus which may significantly narrow or even block the Minimum Lumen Area of the coronary artery. As a result, where the MLA is narrow, a TCFA rupture and the corresponding buildup of thrombus at that location is also more likely to produce an undesirable event.

The definition of a TCFA given above is a pathological definition. However, this pathological definition is not practical for VH-IVUS imaging since the current resolution for VH-IVUS is about 200 microns. Since a TCFA is usually pathologically defined as having a cap thickness of about 65 microns, this cap thickness would not be detectable by current VH-IVUS. As a result, a VH-TCFA is defined as a fibroatheroma where the necrotic core is equal to or exceeds 10% of the plaque with the appearance of the necrotic core being in direct contact with the vessel lumen for over a 30 degree arc from the center of the lumen for at least three consecutive VH-IVUS images along the length of the artery.

An undesirable event that may occur due to the narrowing or even occlusion of a coronary artery is a so-called Clinical Event. "Clinical Event" could be defined as the patient's death, the patient having a heart attack or the patient having unstable or increasing angina within three years. As a result, it would be highly desirable to be able to detect physiology likely to produce Clinical Events in order to prophylactically administer therapeutic or preventative care to prevent a Clinical Event from occurring.

Another undesirable event that may occur is a so-called Silent Clinical Event. A "Silent Clinical Event" may occur when the plaque ruptures. When a plaque ruptures, thrombus (the accumulation of red blood cells) typically occurs around and downstream from the rupture site. This build up of thrombus at the site of or downstream from the rupture causes a narrowing of the vessel lumen (i.e., a diminishing of the Minimum Lumen Area near the site of the rupture) but not in an amount that manifests itself in a Clinical Event. Even though this narrowing does not manifest itself in a clinically detectable manner (due to not blocking a sufficiently large amount of the coronary artery lumen), this narrowing does makes this site particularly vulnerable to additional ruptures or further narrowing or even occlusion. As a result, although a Silent Clinical Event is not a condition severe enough to warrant classification as a Clinical Event, a Silent Clinical Event is a condition with an increased risk for eventually manifesting itself in a Clinical Event. As a result, it would also be highly desirable to be able to detect physiology producing Silent Clinical Events as well in order to prophylactically administer therapeutic or preventative care to prevent a Clinical Event from occurring.

Intravascular ultrasound ("IVUS") combined with a technique called virtual histology ("VH") has been particularly successful in recognizing the morphology of plaque in vivo (i.e., the amount and composition of plaque in the patient's body). The following systems for detecting and characterizing plaque using IVUS with VH are disclosed in US Pat. Nos. 6,200,268 entitled "VASCULAR PLAQUE CHARACTERIZATION" issued March 13, 2001 with D. Geoffrey Vince, Barry D. Kuban and Anuja Nair as inventors, 6,381,350 entitled "INTRAVASCULAR ULTRASONIC ANALYSIS USING ACTIVE CONTOUR METHOD AND SYSTEM" issued April 30, 2002 with Jon D. Klingensmith, D. Geoffrey Vince and Raj Shekhar as inventors, 7,074,188 entitled "SYSTEM AND METHOD OF CHARACTERIZING VASCULAR TISSUE" issued July 11, 2006 with Anuja Nair, D. Geoffrey Vince, Jon D. Klingensmith and Barry D. Kuban as inventors, 7,175,597 entitled "NON-INVASIVE TISSUE CHARACTERIZATION SYSTEM AND METHOD" issued February 13, 2007 with D. Geoffrey Vince, Anuja Nair and Jon D. Klingensmith as inventors, 7,215,802 entitled "SYSTEM AND METHOD FOR VASCULAR BORDER DETECTION" issued May 8, 2007 with Jon D. Klingensmith, Anuja Nair, Barry D. Kuban and D. Geoffrey Vince as inventors, 7,359,554 entitled "SYSTEM AND METHOD FOR IDENTIFYING A VASCULAR BORDER" issued April 15, 2008 with Jon D. Klingensmith, D. Geoffrey Vince, Anuja Nair and Barry D. Kuban as inventors and 7,463,759 entitled "SYSTEM AND METHOD FOR VASCULAR BORDER DETECTION" issued December 9, 2008 with Jon D. Klingensmith, Anuja Nair, Barry D. Kuban and D. Geoffrey Vince, as inventors.

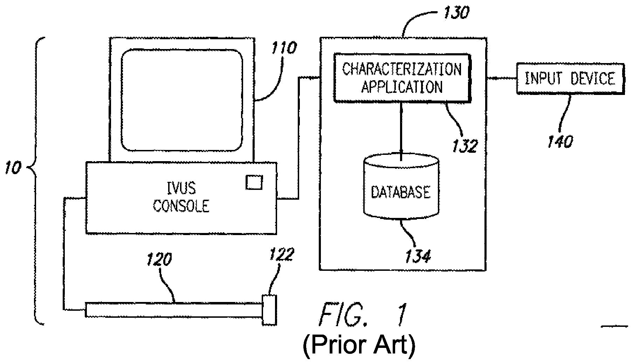

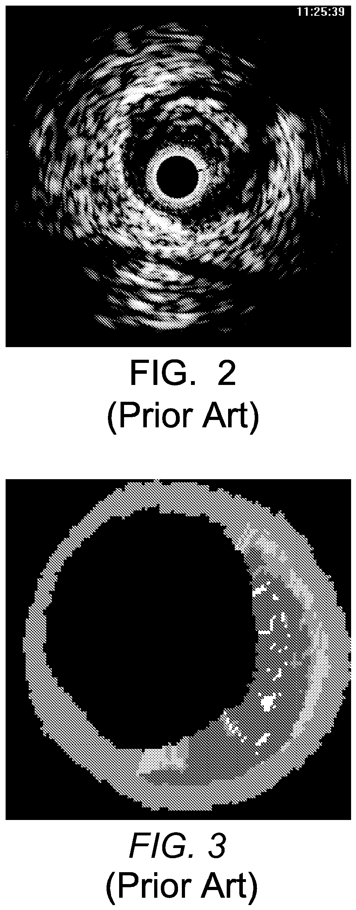

FIG. 1 illustrates a typical tissue-characterization system 10 using IVUS and VH (so called "VH-IVUS"). In the typical tissue - characterization system 10, an intravascular ultrasound (IVUS) console 110 is electrically connected to an IVUS catheter 120 and used to acquire RF backscattered data (i.e., IVUS data) from a blood vessel. The VH-IVUS console 110 typically includes a computing device 130 comprising a database 134 and a characterization application 132 electrically connected to the database 134 and adapted to receive IVUS data from the IVUS console 110 or directly from a transducer 122. Specifically, a transducer 122 is attached to the end of the catheter 120 and is carefully maneuvered through a patient's arteries to a point of interest along the artery. The transducer is then pulsed to acquire high-frequency sonic echoes or backscattered signals reflected from the tissue of the vascular object. Because different types and densities of tissue absorb and reflect the ultrasound pulse differently, the reflected data (i.e., IVUS data) is used to image the vascular object. In other words, the IVUS data can be used (e.g., by the IVUS console 110 or a separate computing device 130) to create an IVUS image. An exemplary IVUS image 20 is shown in FIG. 2, where the light and dark regions indicate different tissue types and/or densities. It should be appreciated that the IVUS console 110 depicted herein is not limited to any particular type of IVUS console, and includes all ultrasonic devices known to those skilled in the art (e.g., a Revolution® or EagleEye® IVUS catheter used in conjunction with an s5™ IVUS imaging system, all of which are sold by Volcano Corporation of San Diego, California). It should further be appreciated that the IVUS catheter 120 depicted herein is not limited to any particular type of catheter, and includes all ultrasonic catheters known to those skilled in the art. Thus, for example, a catheter having a single transducer (e.g., adapted for rotation) or an array of transducers (e.g., circumferentially positioned around the catheter or longitudinally along the catheter 120) can be used with the typical tissue - characterization system 10.

It should be appreciated that the database 134 depicted herein includes, but is not limited to, RAM, cache memory, flash memory, magnetic disks, optical disks, removable disks, SCSI disks, IDE hard drives, tape drives and all other types of data storage devices (and combinations thereof, such as RAID devices) generally known to those skilled in the art. It should further be appreciated that the characterization application 132, as depicted and discussed herein, may exist as a single application or as multiple applications, locally and/or remotely stored. It should also be appreciated that the number and location of the components depicted in FIG. 1 do not limit a typical tissue - characterization system 10 but are merely provided to illustrate a typical tissue - characterization system 10. Thus, for example, a computing device 130 having a plurality of databases 134 or a remotely located characterization application 132 (either in part or in whole) or any combination of these may also be found in a typical tissue - characterization system 10.

In one embodiment of a typical tissue - characterization system 10, the characterization application 132 is adapted to receive and store characterization data (e.g., tissue type, etc.). The characterization data was determined prior to using the tissue - characterization system 10 as follows. After a specimen vascular object has been interrogated (e.g., IVUS data has been collected), a histology correlation is prepared. In other words, the specimen vascular object is dissected or cross-sectioned for histology. In one method of producing characterization data, the cross-section is previously marked, for example with a suture, so that the histology can be corresponded to a portion of the IVUS image. The cross-section is then prepared with a fixing and staining process that is well known in the art. The staining process allows a clinician to identify a tissue type(s), or a chemical(s) found within (e.g., a chemical corresponding to a particular tissue type, etc.). The identified tissue type or types is then correlated to the IVUS data as will be explained below.

It should be appreciated that there may be many methods used to identify or characterize the cross-sectional object as is well understood in the art besides the method just described. Thus, any identification/characterization method generally known to those skilled in the art may be used to characterize tissue. The identified tissue type or characterization (i.e., characterization data) is then provided to the characterization application 132. In one embodiment, as shown in FIG. 1, the characterization data is provided via an input device 140 electrically connected to the computing device 130. The characterization data is preferably then stored in the database 134. It should be appreciated that the input device depicted herein includes, but is not limited to, a keyboard, a mouse, a scanner and all other data-gathering and/or data-entry devices generally known to those skilled in the art. It should further be appreciated that the term tissue type or characterization, as these terms are used herein, include, but are not limited to, fibrous tissues, fibro-lipidic tissues, calcified necrotic tissues, calcific tissues, collagen compositions, cholesterol, thrombus, compositional structures (e.g., the lumen, the vessel wall, the medial-adventitial boundary, etc.) and all other identifiable characteristics generally known to those skilled in the art.

In one method of characterizing tissue, the characterization application 132 is adapted to create a VH image where specifically characterized tissue is associated with its corresponding region on an IVUS image. Specifically, digitized data is provided to the characterization application 132 (e.g., via the IVUS catheter 120 where the digitized data corresponds to the physiology of the patient's blood vessel. The characterization application 132 uses data from the database 134 to characterize tissue corresponding to the digitized data from the IVUS catheter 120 and create a VH image (i.e., a digital image or outline that substantially corresponds to the vascular object and where specific tissue types are identified). A region of interest (ROI) on the VH image can then be identified by the operator. Preferably, the ROI is characterized by the characterization data, as previously provided, and may be the entire VH image or a portion thereof. The characterization application 132 is then adapted to identify a corresponding region (e.g., x,y coordinates, etc.) on the IVUS image where the corresponding region may be specifically identified (e.g., by coloring the corresponding region with a pre-determined color).

In view of the foregoing, what is needed is an effective device and method for alerting a healthcare provider with an indication that a patient's physiology makes the patient more likely to experience a Clinical Event or a Silent Clinical Event.

### Summary of the Invention

In accordance with embodiments, a patient's physiological parameters are ascertained using standard intravascular ultrasound (IVUS) in combination with virtual histology (VH) and then these physiological parameters are evaluated to predict whether the patient's physiology is at clinically significant increased risk to produce a Clinical Event or a Silent Clinical Event compared to the population at large. In particular, in one embodiment the following three physiological parameters: the Plaque Burden; the Minimum Lumen Area; and whether a VH-TCFA or multiple VH-TCFAs are present are compared to target values and ranges. A concurrence of the values for the physiological parameters falling within the target ranges indicates that a patient's physiology is at an increased risk to produce a Clinical Event or a Silent Clinical Event, respectively than the population at large. In other embodiments, combinations of any two of the three physiological parameters listed above are compared to target values and ranges. A concurrence of the values for two of the physiological parameters falling within the target ranges indicates that a patient's physiology is also at an increased risk to produce a Clinical Event than the population at large. In still other embodiments, any of the three physiological parameters listed above are compared to target values and ranges. Any of the values of the physiological parameters falling within the target ranges indicates that a patient's physiology is at an increased risk to produce a Clinical Event than the population at large.

It is an object of this invention in one or more embodiments to ascertain whether a patient's physiology puts the patient at an increased risk of experiencing a Silent Clinical Event.

This object is solved with a device for determining whether a patient's physiology puts him or her at an increased risk of experiencing a Silent Clinical Event according to the invention comprising the features of claim 1.

The invention will be described hereafter in detail with particular reference to the drawings. Throughout this description, like elements, in whatever embodiment described, refer to common elements wherever referred to and referenced by the same reference number. The characteristics, attributes, functions, interrelations ascribed to a particular element in one location apply to that element when referred to by the same reference number in another location unless specifically stated otherwise.

### Brief Descriptions of the Drawings

Figure 1 is a schematic view of a typical tissue - characterization system using IVUS and VH.
Figure 2 is an exemplary IVUS image.
Figure 3 is an exemplary VH image.

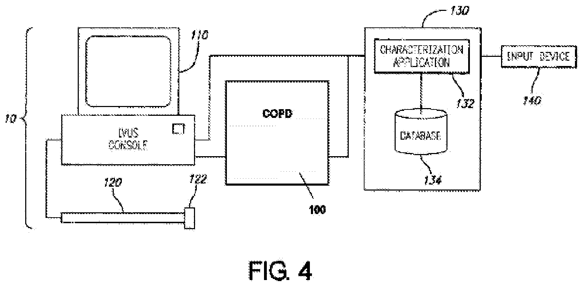

Figure 4 is a schematic view of an embodiment of the present invention applied to the tissue - characterization system of Figure 1.
Figure 5 is a flowchart showing the algorithm of one embodiment of the invention.
Figure 6 is a Venn diagram associated with the algorithm of Figure 5 showing the concurrence of conditions needed to produce the condition "Increased Risk of Clinical Event."
Figure 7 is a flowchart showing the algorithm of another embodiment of the invention.
Figure 8 is a Venn diagram associated with the algorithm of Figure 7 showing the concurrence of conditions needed to produce the condition "Increased Risk of Silent Clinical Event."
Figure 9 is a flowchart showing the algorithm of another embodiment of the invention.
Figure 10 is a Venn diagram associated with the algorithm of Figure 9 showing the concurrence of conditions needed to produce the condition "Increased Risk of Clinical Event."
Figure 11 is a flowchart showing the algorithm of another embodiment of the invention.
Figure 12 is a Venn diagram associated with the algorithm of Figure 11 showing the concurrence of conditions needed to produce the condition "Increased Risk of Clinical Event."
Figure 13 is a flowchart showing the algorithm of another embodiment.
Figure 14 is a Venn diagram associated with the algorithm of Figure 13 showing the concurrence of conditions needed to produce the condition "Increased Risk of Clinical Event."
Figure 15 is a flowchart showing the algorithm of another embodiment.
Figure 16 is a Venn diagram associated with the algorithm of Figure 15 showing the condition needed to produce the condition "Increased Risk of Clinical Event."
Figure 17 is a flowchart showing the algorithm of another embodiment.
Figure 18 is a Venn diagram associated with the algorithm of Figure 17 showing the condition needed to produce the condition "Increased Risk of Clinical Event."
Figure 19 is a flowchart showing the algorithm of another embodiment.
Figure 20 is a Venn diagram associated with the algorithm of Figure 19 showing the condition needed to produce the condition "Increased Risk of Clinical Event."

### Description of the Preferred Embodiments

Referring to the Figures, a Clinical Outcome Predictor Device (COPD) is shown in the Figures generally referred to by the reference number 100. The COPD 100 includes or may be an adjunct to the tissue-characterization system 10 as described above including a console 110, IVUS catheter 120, a computing device 130 comprising a database 134 and a characterization application 132 electrically connected to the database 134 and typically run on the computing device 130. The COPD 100 is adapted to communicate, that is both receive and transmit data and information, from the IVUS console 110 or the computing device 130 or both. The COPD 100 is preferably operated on the computing device 130 but may also be operated separately and operatively
connected to the console 110, computing device 130, characterization application 132 or database 134 or any combination of these.

The COPD 100 is used to determine whether a patient's physiology indicates that the patient has an increased risk of having a Clinical Event or a Silent Clinical Event compared to the population at large. By increased risk we mean that the Hazard Ratio exceeds 2.0. The Hazard Ratio is a measure of how often a particular event happens in one group compared to how often it happens in another group, over time. A Hazard Ratio of one here means that there is no difference in the occurrence of a Clinical Event or Silent Clinical Event between the two groups. A Hazard Ratio of greater than two means that occurrence of a Clinical Event or Silent Clinical Event was significantly more likely in one of the groups. Here, the control group is the population at large and the comparison group is the group with the particular physiology ascribed to each method and device described hereafter. The higher the Hazard Ratio, the more likely patient's having the particular physiology are to have a Clinical Event or Silent Clinical Event as compared to the population at large.

The tissue - characterization system 10 is used to ascertain the Plaque Burden of the patient's target coronary artery, the Minimum Lumen Area of that coronary artery and whether a VH-TCFA is present in that coronary artery according to standard processes and algorithms as is well understood by those skilled in the art. That data is typically stored in the database 134 to be used as will be described hereafter.

The COPD 100 then evaluates the ascertained Plaque Burden, Minimum Lumen Area and whether a VH-TCFA is present to determine whether the patient's physiology is at an increased risk to have a Clinical Event. In a preferred embodiment of the COPD 100, the COPD 100 is a software application run on the computing device 130.

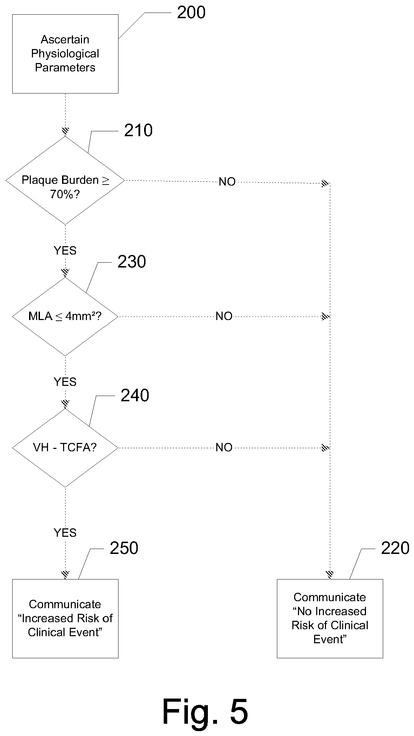

One method of performing this evaluation is by running an algorithm on the COPD 100 as will be described hereafter. Figure 5 is a flowchart illustrating an example of the algorithm. In Figure 5 at step 200, values for the Plaque Burden, Minimum Lumen Area and whether a VH-TCFA is present are determined by the use of the tissue - characterization system 10 as described above. The program then passes to step 210 where the ascertained value for the Plaque Burden is compared to an established or target range of Plaque Burdens. The established or target range is about 70% to 100%. This range represents a Plaque Burden where plaque substantially to completely occludes the lumen of the artery. If the ascertained Plaque Burden does not fall within this range, the program passes to step 220 where the determination that the physiological parameter is not within the target range, and thus that the patient's physiology puts the patient less at risk to have a Clinical Event, is communicated to the healthcare provider.

If the ascertained Plaque Burden falls within this target range, the program passes to step 230 where the ascertained Minimum Lumen Area of interest is compared to predetermined or target Minimum Lumen Areas. The target Minimum Lumen Area in step 230 is less than or equal to about 4 mm². This target Minimum Lumen Area represents a relatively small open lumen in the arterial section of interest. Since these coronary arteries have relatively small Minimum Lumen Areas at least at the point where there is a relatively high Plaque Burden, they are more likely to be affected by the presence of necrotic core, thrombus or other materials capable of occluding the coronary artery lumen. Coronary arteries with relatively larger Minimum Lumen Areas are less likely to be occluded in a clinically significant way by the presence of these occluding entities. If the ascertained Minimum Lumen Area does not fall within this range, the program passes to step 220.

If the Minimum Lumen Area of the coronary artery falls within the target range, the program passes to step 240 where it is determined whether a VH-TCFA is present. If a VH-TCFA is not present, as ascertained by the tissue - characterization system 10, the program passes to step 220. If a VH-TCFA is present, the program passes to step 250.

In order for the program to have progressed to step 250, all three ascertained physiological parameters have had to have values falling within the target ranges. This indicates that the patient's physiological condition has an increased risk of manifesting itself in a Clinical Event. Step 250 communicates this determination to the healthcare provider so that appropriate medical response can be undertaken.

Likewise, as mentioned above, step 220 is reached if any physiological parameter of interest does not fall within the target range. This indicates that the patient's physiological condition does not have the increased risk of manifesting itself in a Clinical Event that the concurrence of these parameters falling within their target ranges produces. As a result, this information should be communicated to the healthcare provider as well.

In the algorithm described above, the steps of comparing the Plaque Burden, Minimum Lumen Area and whether a VH-TCFA is present have been described as being performed in a specific order. However, for this embodiment of the invention, these steps may be performed in any order.

In practice, the values for the Plaque Burden, Minimum Lumen Area and whether a VH-TCFA is present vary with location along a particular artery of interest. Data for determining these values is gathered by the tissue - characterization system 10 by inserting the IVUS catheter 120 into the artery of interest and then pulling the IVUS catheter 120 back while gathering data. Each bit of gathered data is specific to a particular location along the artery of interest. In order for there to be a determination that there is an a Increased Risk of Clinical Event, for at least one location along the artery of interest, all three "targets" must be met for at least that location. In other words, for there to be a determination that the patient's physiology puts him or her at an increased risk for a Clinical Event, for at least one location along the artery of interest, the Plaque Burden must be equal to or greater than about 70%, the Minimum Lumen Area has to be less than or equal to about 4 mm² and a VH-TCFA has to be present.

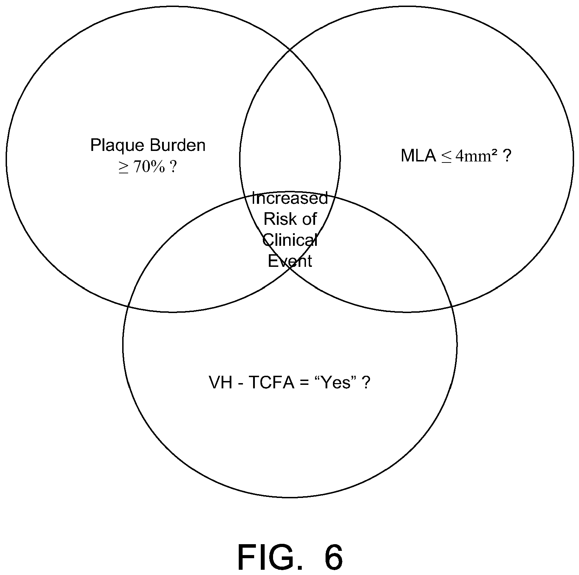

As a result, the determination of whether the patient's physiology puts him or her at an increased risk for a Clinical Event can be thought of in the context of a Venn diagram (Figure 6) where the condition "Increased Risk of Clinical Event" is the concurrence of the Plaque Burden being equal to or greater than about 70%, the Minimum Lumen Area being less than or equal to about 4 mm² and a VH-TCFA is present. Although a specific implementation of determining whether the condition "Increased Risk of Clinical Event" has been described above, it is intended that any system for determining whether the condition "Increased Risk of Clinical Event" is present by the concurrence of these physiological parameters meeting the stated "target" ranges or values is intended to fall within the scope of the invention.

In the embodiment of the invention described above, an algorithm was used only to determine whether the patient's physiological parameters were in a state with an increased risk of manifesting itself in a Clinical Event. In an alternate embodiment of the invention, the COPD 100 is used to detect whether a patient's physiology puts the patient at an increased risk of having a Clinically Silent Event either singly or in combination with detecting physiology putting the patient at an increased risk of having a Clinical Event.

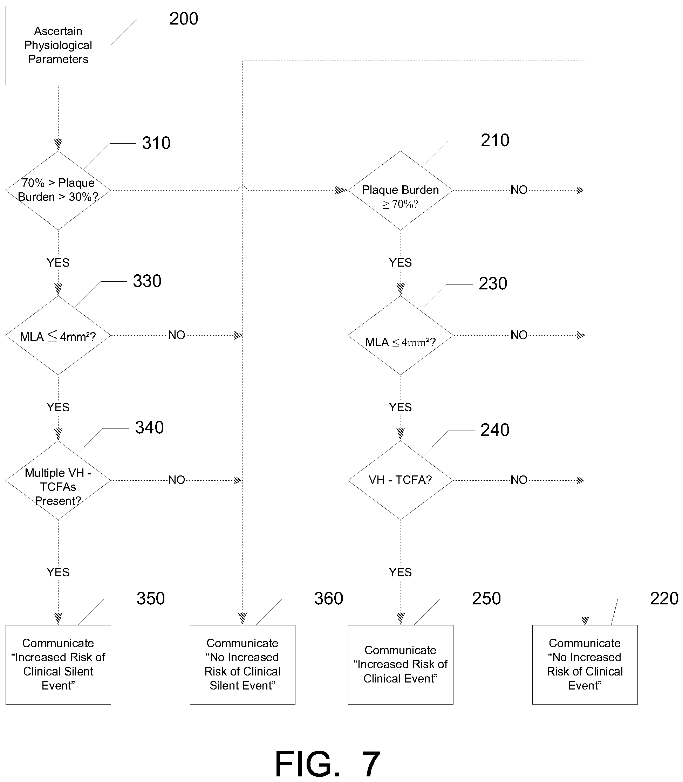

One method of performing this evaluation is by running an algorithm on the COPD 100. Figure 7 is a flowchart illustrating such an algorithm. In Figure 7, values for the Plaque Burden, Minimum Lumen Area and whether a VH-TCFA is present are determined by the use of the tissue - characterization system 10 in step 200. Again, these values for the Plaque Burden of the patient's target coronary artery, the Minimum Lumen Area of that coronary artery and whether a VH-TCFA is present in that coronary artery are ascertained according to standard processes and algorithms as is well understood by those skilled in the art. That data is stored in the database 134 to be used as will be described hereafter.

The program then passes to step 310 where the ascertained value for the Plaque Burden is compared to an established or target range of Plaque Burdens. The established or target range is between about 30% and about 70%. This range represents a Plaque Burden where plaque is beginning to occlude the lumen of the artery but is not yet significantly occluding the artery. If the ascertained Plaque Burden does not fall within this range, the program passes to step 210 where an analysis of whether the patient's physiology puts the patient at an increased risk of experiencing a Clinical Event is performed as described above. If, at step 210 it is determined that the patient's Plaque Burden is not equal to or greater than 70%, this indicates that the patient's Plaque Burden is equal to or less than about 30%. As a result, the program progresses from step 210 to both steps 220 and 360 as will be explained below. The outcomes of the analysis performed by steps 210, 230 and 240 in the embodiment shown in Figure 7 is either that the patient's physiology puts the patient at an increased risk of experiencing a Clinical Event (step 250) or, as communicated at step 220, that the patient is not at an increased risk of experiencing a Clinical Event that the concurrence of parameters falling with in target values or steps 210, 230 and 240 require.

If, at step 310, the ascertained Plaque Burden falls within the 30% to 70% target range, the program passes to step 330 where the ascertained Minimum Lumen Area of the coronary artery of interest is compared to predetermined or target Minimum Lumen Area of that coronary artery. The target Minimum Lumen Area in step 330 is again less than or equal to about 4 mm². Minimum Lumen Areas larger than about 4 mm² represent relatively large coronary arteries. Since such coronary arteries have relatively large Minimum Lumen Area, they are less likely to be affected by the presence of necrotic core, thrombus or other materials capable of occluding the coronary artery lumen as compared to coronary arteries with smaller Minimum Lumen Areas as described above. If the ascertained coronary Minimum Lumen Area does not fall within this range (i.e., the Minimum Lumen Area is larger than about 4 mm²), the program passes to step 360 which indicates that the patient's physiological condition does not have the increased risk of manifesting itself in a Silent Clinical Event that the concurrence of these parameters falling within their target ranges produces.

If the Minimum Lumen Area of the coronary artery falls within the target range (i.e., the Minimum Lumen Area is smaller than about 4 mm²), the program passes to step 340 where it is determined whether multiple VH-TCFAs are present. If multiple VH-TCFAs are not present, as ascertained by the tissue - characterization system 10, the program passes to step 360. If multiple VH-TCFAs are present the program passes to step 350.

In order for the program to have progressed to step 350, all three ascertained physiological parameters have had to have values falling within the target ranges. This indicates that the patient's physiological condition is in the condition called "Silent Clinical Event." Step 350 communicates this determination to the healthcare provider so that appropriate medical response can be undertaken.

Likewise, step 360 is reached if any physiological parameter of interest does not fall within the target range for indicating that the patient's physiology does not have the increased risk of manifesting itself in a Silent Clinical Event that the concurrence of these parameters falling within their target ranges produces. This indicates that the patient's physiological condition does not have an increased risk of manifesting itself in a Silent Clinical Event. As a result, this information should be communicated to the healthcare provider as well.

Again, in practice, the values for the Plaque Burden, Minimum Lumen Area and whether a VH-TCFA is present vary with location along a particular artery of interest. Data for determining these values is gathered by the tissue - characterization system 10 by inserting the IVUS catheter 120 into the artery of interest and then pulling the IVUS catheter 120 back while gathering data. Each bit of gathered data is specific to a particular location along the artery of interest. In order for there to be a determination that a patient's physiology puts the patient at an increased risk of Silent Clinical Event, for at least one location along the artery of interest, all three "targets" must be met. In other words, for there to be a determination that there is an increased risk of Silent Clinical Event, for at least one location along the artery of interest, the Plaque Burden must be between about 30% and about 70%, the Minimum Lumen Area has to be less than or equal to about 4 mm² and multiple VH-TCFAs have to be present.

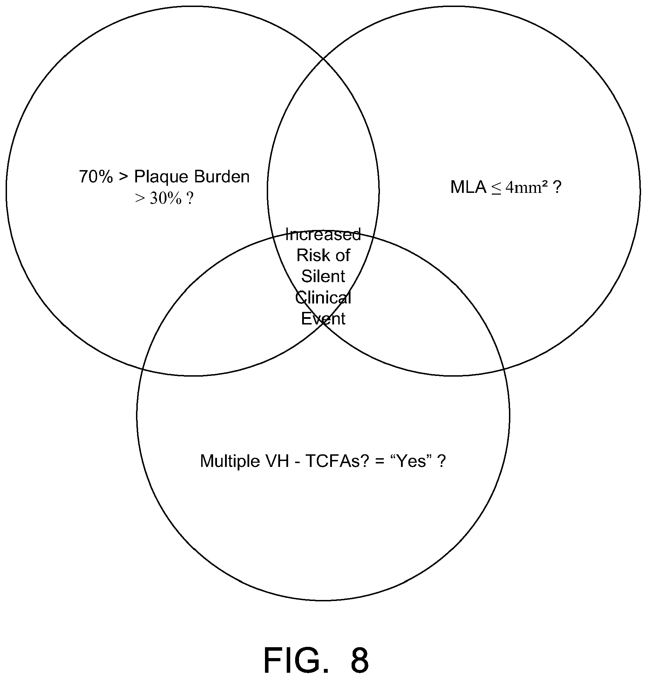

As a result, the determination of whether there is an increased risk of Silent Clinical Event can be thought of in the context of a Venn diagram (Figure 8) where the condition "Increased Risk of Silent Clinical Event" is the concurrence of the Plaque Burden being between about 30% to about 70%, the Minimum Lumen Area being less than or equal to about 4 mm² and multiple VH-TCFAs being present. Although a specific implementation of determining whether the condition "Increased Risk of Silent Clinical Event" has been described above, it is intended that any system for determining whether the condition "Increased Risk of Silent Clinical Event" is present by the concurrence of these physiological parameters meeting the stated "target" ranges or values is intended to fall within the scope of the invention.

Further, the determination of whether there is an "Increased Risk of Silent Clinical Event" may be determined in a variant of this algorithm separately of a determination of whether there is an "Increased Risk of Clinical Event." Determining whether there is an "Increased Risk of Silent Clinical Event" separately of a determining whether there is an "Increased Risk of Clinical Event" may easily be done by removing the steps on Figure 7 as described above related to determining whether there is an "Increased Risk of Clinical Event."

In the embodiments of the invention described above, a concurrence of the three physiological parameters: the Plaque Burden; the Minimum Lumen Area; and whether there is a VH-TCFA or multiple VH-TCFAs are compared to target values and ranges. As explained, a concurrence of the values for the physiological parameters falling within the target ranges indicates that a patient's physiology puts the patient at increased risk for having a Clinical Event or a Silent Clinical Event, respectively. However, it is also within the scope of the present invention for combinations of any two of the three physiological parameters listed above to be individually compared to target values and ranges.

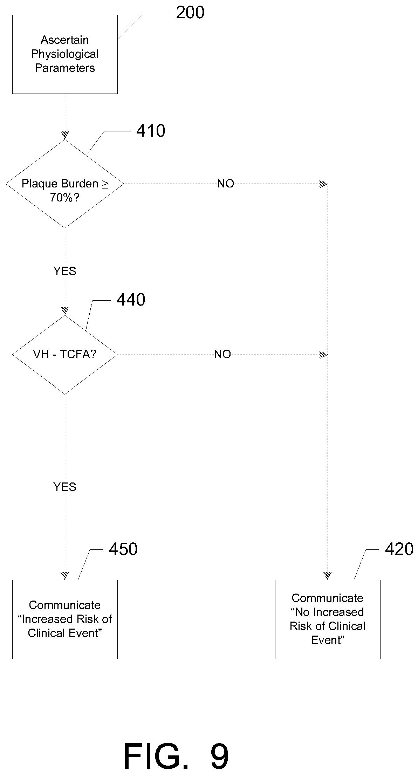

In particular, another method for determining whether there is an increased likelihood of having a Clinical Event is performed by running an algorithm on the COPD 100 as will be described hereafter. Figure 9 is a flowchart illustrating an example of the algorithm. In Figure 9 at step 200, values for the following patient physiological parameters: the Plaque Burden and whether a VH-TCFA is present are determined by the use of the tissue - characterization system 10 as described above. The program then passes to step 410 where the ascertained value for the Plaque Burden is compared to an established or target range of Plaque Burdens. The established or target range is about 70% to 100%. This range represents a Plaque Burden where plaque substantially to completely occludes the lumen of the artery. If the ascertained Plaque Burden does not fall within this range, the program passes to step 420 where the determination that the physiological parameter is not within the target range, and thus that the patient's physiology does not the patient at an increased risk to have a Clinical Event, is communicated to the healthcare provider.

If, at step 410, the ascertained Plaque Burden falls within this target range, the program passes to step 440 where it is determined whether a VH-TCFA is present. If a VH-TCFA is not present, the program passes to step 420. If a VF-TCFA is present, the program passes to step 450.

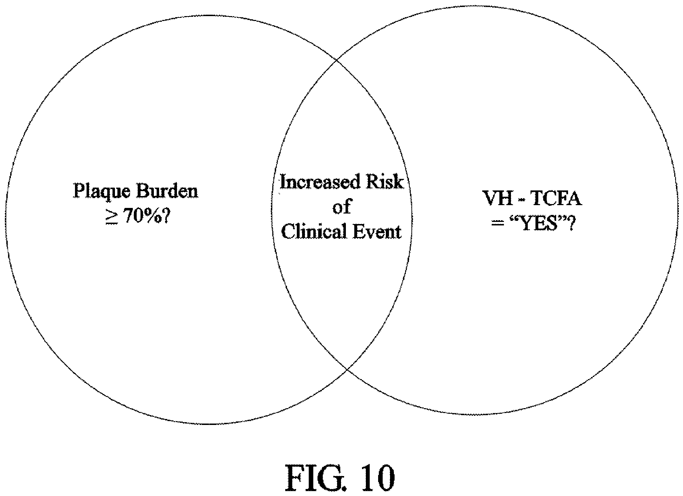

In order for the program to have progressed to step 450, both ascertained physiological parameters have had to have values falling within the target ranges (Figure 10). This indicates that the patient's physiological condition has an increased risk of manifesting itself in a Clinical Event. Step 450 communicates this determination to the healthcare provider so that an appropriate medical response can be undertaken.

Likewise, as mentioned above, step 420 is reached if any physiological parameter of interest does not fall within the target range. This indicates that the patient's physiological condition does not have a significantly increased risk of manifesting itself in a Clinical Event. As a result, this information should be communicated to the healthcare provider as well.

In the algorithm described above, the steps of comparing the Plaque Burden and whether a VH-TCFA is present have been described as being performed in a specific order. However, for this embodiment of the invention, these steps may be performed in any order.

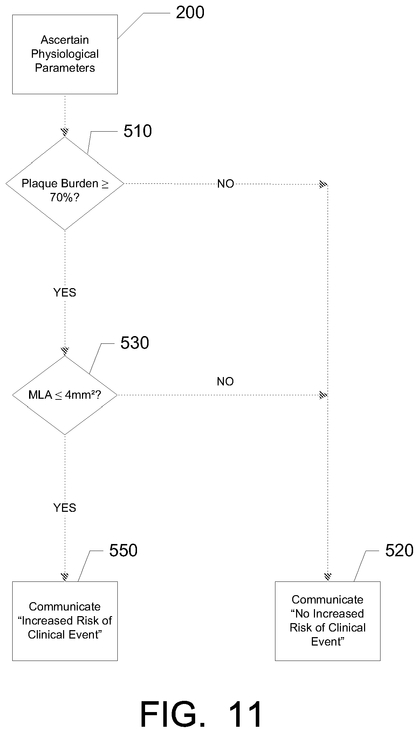

Another method for determining whether there is an increased risk of having a Clinical Event is performed by running an algorithm on the COPD 100 as will be described hereafter. Figure 11 is a flowchart illustrating an example of the algorithm. In Figure 11 at step 200, values for the following patient physiological parameters: the Plaque Burden and Minimum Lumen Area are determined by the use of the tissue - characterization system 10 as described above. The program then passes to step 510 where the ascertained value for the Plaque Burden is compared to an established or target range of Plaque Burdens. The established or target range is about 70% to 100%. This range represents a Plaque Burden where plaque substantially to completely occludes the lumen of the artery. If the ascertained Plaque Burden does not fall within this range, the program passes to step 520 where the determination that the physiological parameter is not within the target range, and thus that the patient's physiology does not put the patient at an increased risk to have a Clinical Event, is communicated to the healthcare provider

If the ascertained Plaque Burden falls within this target range, the program passes to step 530 where the ascertained Minimum Lumen Area of interest is compared to predetermined or target Minimum Lumen Areas. The target Minimum Lumen Area in step 530 is less than or equal to about 4 mm². This target Minimum Lumen Area represents a relatively small open lumen in the arterial section of interest. Since these coronary arteries have relatively small Minimum Lumen Areas, at least at the point where there is a relatively high Plaque Burden, they are more likely to be affected by the presence of necrotic core, thrombus or other materials capable of occluding the coronary artery lumen. Coronary arteries with relatively larger Minimum Lumen Areas are less likely to be occluded in a clinically significant way by the presence of these occluding entities. If the ascertained Minimum Lumen Area does not fall within this range, the program passes to step 520. If the Minimum Lumen Area of the coronary artery falls within the target range, the program passes to step 550.

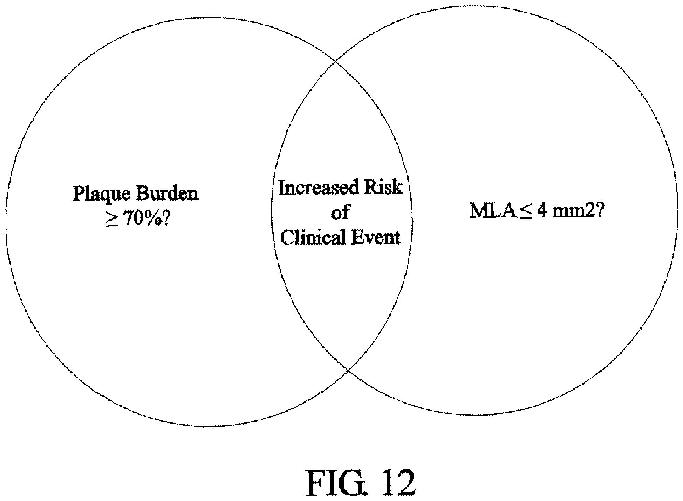

In order for the program to have progressed to step 550, both ascertained physiological parameters have had to have values falling within the target ranges (Figure 12). This indicates that the patient's physiological condition has an increased risk of manifesting itself in a Clinical Event. Step 550 communicates this determination to the healthcare provider so that appropriate medical response can be undertaken.

Likewise, as mentioned above, step 520 is reached if any physiological parameter of interest does not fall within the target range. This indicates that the patient's physiological condition does not have put the patient at an increased risk of manifesting itself in a Clinical Event. As a result, this information should be communicated to the healthcare provider as well.

In the algorithm described above, the steps of comparing the Plaque Burden and Minimum Lumen Area have been described as being performed in a specific order. However, for this embodiment of the invention, these steps may be performed in any order.

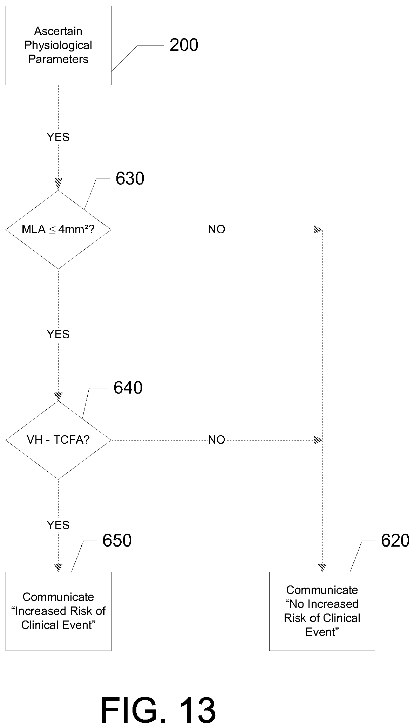

Yet another method for determining whether there is an increased likelihood of having a Clinical Event is performed by running an algorithm on the COPD 100 as will be described hereafter. Figure 13 is a flowchart illustrating an example of the algorithm. In Figure 13 at step 200, values for the following patient physiological parameters: the Minimum Lumen Area and whether a VH-TCFA is present are determined by the use of the tissue - characterization system 10 as described above. The program then passes to step 630 where the ascertained value for the Minimum Lumen Area is compared to an established or target range of Minimum Lumen Areas. The established or target range is about less than or equal to about 4 mm². If the ascertained Minimum Lumen Area does not fall within this range, the program passes to step 620 where the determination that the physiological parameter is not within the target range, and thus that the patient's physiology does not put the patient at an increased risk to have a Clinical Event, is communicated to the healthcare provider.

If the ascertained Minimum Lumen Area falls within this target range, the program passes to step 640 where it is determined whether a VH-TCFA is present. If a VH-TCFA is not present, the program passes to step 620. If a VH-TCFA is present, the program passes to step 650.

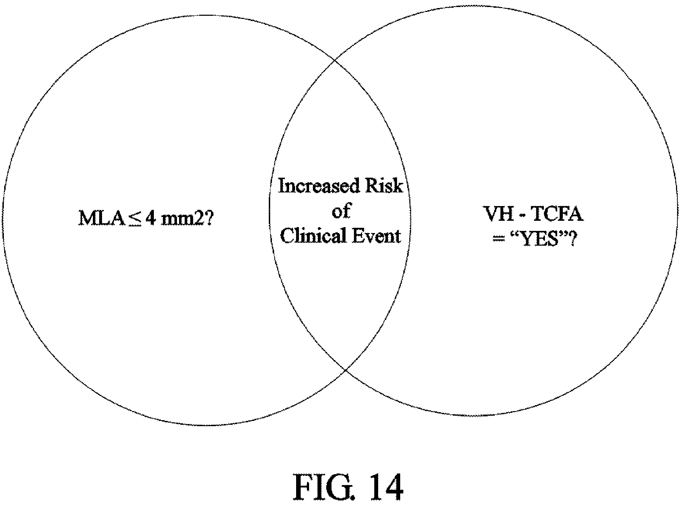

In order for the program to have progressed to step 650, both ascertained physiological parameters have had to have values falling within the target ranges (Figure 14). This indicates that the patient's physiological condition has an increased risk of manifesting itself in a Clinical Event. Step 650 communicates this determination to the healthcare provider so that appropriate medical response can be undertaken.

Likewise, as mentioned above, step 620 is reached if any physiological parameter of interest does not fall within the target range. This indicates that the patient's physiological condition does not have a significantly increased risk of manifesting itself in a Clinical Event. As a result, this information should be communicated to the healthcare provider as well. In the algorithm described above, the steps of comparing the Minimum Lumen Area and determining whether a VH-TCFA is present have been described as being performed in a specific order. However, for this embodiment of the invention, these steps may be performed in any order.

In the embodiments of the invention described above, various combinations of two out of the three physiological parameters: the Plaque Burden; the Minimum Lumen Area; and whether a VH-TCFA or multiple VH-TCFAs are present and compared to target values and ranges and concurrences of any two of these physiological parameters with their corresponding target ranges indicates that a patient's physiology is likely to produce a Clinical Event or a Silent Clinical Event, respectively. However, it is also within the scope of the present invention for any of the three physiological parameters listed above to be compared to target values and ranges.

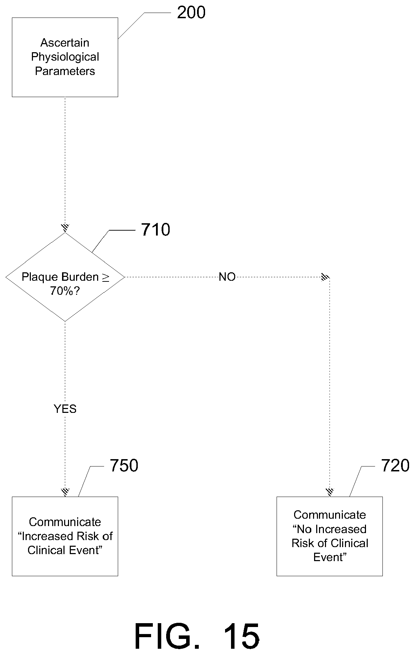

Consequently, another method for determining whether there is an increased likelihood of having a Clinical Event is performed by running an algorithm on the COPD 100 as will be described hereafter. Figure 15 is a flowchart illustrating an example of the algorithm. In Figure 15 at step 200, the value for the Plaque Burden is determined by the use of the tissue - characterization system 10 as described above. The program then passes to step 710 where the ascertained value for the Plaque Burden is compared to an established or target range of Plaque Burdens. The established or target range is about 70% to 100%. If the ascertained Plaque Burden does not fall within this range, the program passes to step 720 where the determination that the physiological parameter is not within the target range, and thus that the patient's physiology does not put the patient at an increased risk to have a Clinical Event, is communicated to the healthcare provider. If the ascertained Plaque Burden falls within this target range, the program passes to step 750.

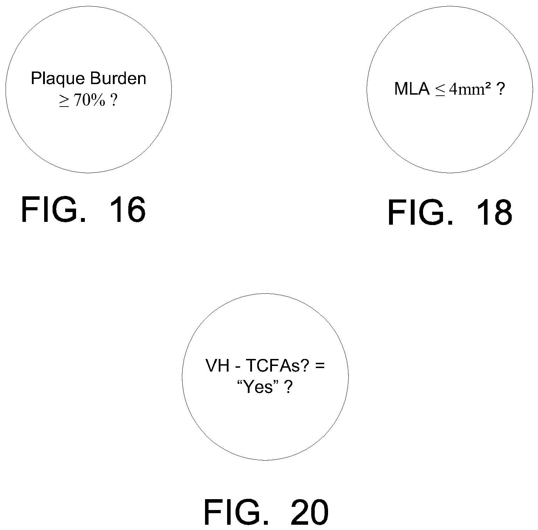

In order for the program to have progressed to step 750, the value ascertained for the Plaque Burden must have had a value falling within the target range (Figure 16). This indicates that the patient's physiological condition does not put the patient at an increased risk of manifesting itself in a Clinical Event. Step 750 communicates this determination to the healthcare provider so that appropriate medical response can be undertaken.

Likewise, as mentioned above, step 720 is reached if the plaque burden does not fall within the target range. This indicates that the patient's physiological condition does not put the patient at an increased risk of manifesting itself in a Clinical Event. As a result, this information should be communicated to the healthcare provider as well

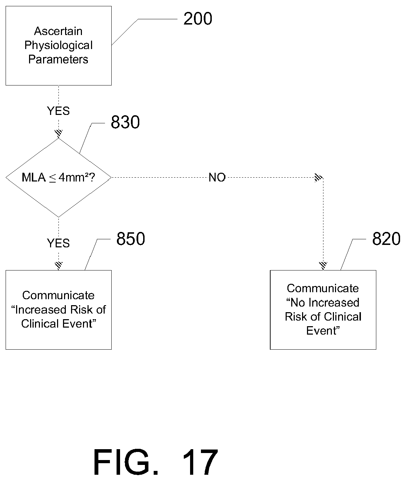

Yet a further method determining the increased likelihood of having a Clinical Event is performed by running an algorithm on the COPD 100 as will be described hereafter. Figure 17 is a flowchart illustrating an example of the algorithm. In Figure 17 at step 200, the value for the Minimum Lumen Area is determined by the use of the tissue - characterization system 10 as described above. The program then passes to step 830 where the ascertained value for the Minimum Lumen Area is compared to an established or target range of Minimum Lumen Areas. The established or target range is about less than or equal to about 4 mm². If the ascertained Minimum Lumen Area does not fall within this range, the program passes to step 830 where the determination that the physiological parameter is not within the target range, and thus that the patient's physiology does not put the patient at an increased risk to have a Clinical Event, is communicated to the healthcare provider. If the ascertained Minimum Lumen Area falls within this target range, the program passes to step 850.

In order for the program to have progressed to step 850, the Minimum Lumen Area had to have a value falling within the target ranges (Figure 18). This indicates that the patient's physiological condition has an increased risk of manifesting itself in a Clinical Event. Step 850 communicates this determination to the healthcare provider so that appropriate medical response can be undertaken.

Likewise, as mentioned above, step 820 is reached if the Minimum Lumen Area does not fall within the target range. This indicates that the patient's physiological condition does not put the patient at an increased risk of manifesting itself in a Clinical Event. As a result, this information should be communicated to the healthcare provider as well.

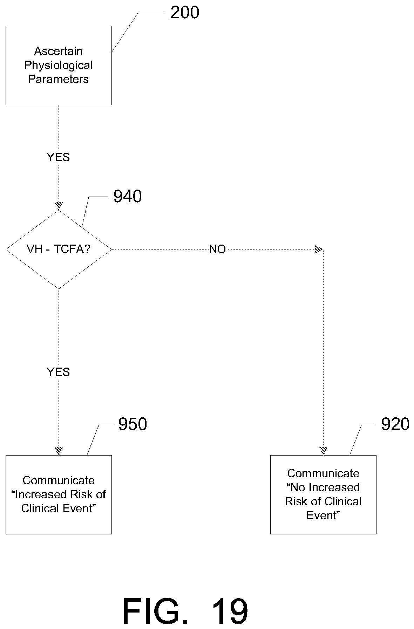

Another method of determining whether there is an increased likelihood of having a Clinical Event is performed by running an algorithm on the COPD 100 as will be described hereafter. Figure 19 is a flowchart illustrating an example of the algorithm. In Figure 19 at step 200, the data necessary to determine whether a VH-TCFA is present is collected by the use of the tissue - characterization system 10 as described above. The program then passes to step 940 where it is determined whether a VH-TCFA is present. If a VH-TCFA is not present, the program passes to step 920 where the determination that the physiological parameter is not within the target range, and thus that the patient's physiology does not put the patient at an increased risk to have a Clinical Event, is communicated to the healthcare provider. If a VH-TCFA is present, the program passes to step 950.

In order for the program to have progressed to step 950, a VH-TCFA must be present (Figure 20). This indicates that the patient's physiological condition has an increased risk of manifesting itself in a Clinical Event. Step 950 communicates this determination to the healthcare provider so that appropriate medical response can be undertaken.

Likewise, as mentioned above, step 920 is reached is a VH-TCFA is not present. This indicates that the patient's physiological condition does not put the patient at an increased risk of manifesting itself in a Clinical Event. As a result, this information should be communicated to the healthcare provider as well.

Steps 250, 450, 550, 650, 750, 850 and 950 communicate to the healthcare provider that the respective parameter or parameters have all fallen within target ranges thereby indicating that the patient's physiology has an increased risk of producing a Clinical Event so that appropriate medical response can be undertaken. The communication in steps 250, 450, 550, 650, 750, 850 and 950 may take the form of a message displayed on console 110, the modifying of an image of the coronary artery displayed on the console 110 such as by appending a text or color indicator that the patient's physiology at that location on the artery is such that the patient is more likely to experience a Clinical Event, the communication of the parameter values themselves separately or as part of one of the previous messages or by any other means well within the skill of one skilled in the art to communicate such a determination.

Likewise, as mentioned above, steps 220, 420, 520, 620, 720, 820 and 920 are reached if any physiological parameter of interest does not fall within the target range. This indicates that the patient's physiological condition does not have an increased risk of manifesting itself in a Clinical Event. As a result, this information should be communicated to the healthcare provider as well. The communication in steps 220, 420, 520, 620, 720, 820 and 920 may also take the form of a message displayed on console 110, the modifying of an image of the coronary artery displayed on the console 110 such as by appending a text or color indicator that the patient's physiology at that location on the artery is such that the patient is not at an increased risk to experience a Clinical Event, the communication of the parameter values themselves separately or as part of one of the previous messages or by any other means well within the skill of one skilled in the art to communicate such a determination.

Step 350 communicates to the healthcare provider that the Plaque Burden, Minimum Lumen Area and that a VH-TCFA or multiple VH-TCFAs are present having values all falling within the target ranges of steps 310, 330 and 340 thereby indicating that the patient's physiology has an increased risk of producing a Silent Clinical Event so that appropriate medical response can be undertaken. The communication in step 350 may take the form of a message displayed on console 110, the modifying of an image of the coronary artery displayed on the console 110 such as by appending a text or color indicator that the patient's physiology at that location on the artery is such that the patient is at an increased risk to experience a Silent Clinical Event, the communication of the parameter values themselves separately or as part of one of the previous messages or by any other means well within the skill of one skilled in the art to communicate such a determination.

Further, as mentioned above, step 360 is reached if the respective physiological parameters are not within the targets of steps 310, 330 and 340, and in the embodiment shown in Figure 7 having steps 210, 230 and 240 are not within the targets of steps 210, 230 and 240, and thus do not fall within the target range. This indicates that the patient's physiological condition does not have an increased risk of manifesting itself in a Silent Clinical Event. As a result, this information should be communicated to the healthcare provider as well. The communication in step 360 may also take the form of a message displayed on console 110, the modifying of an image of the coronary artery displayed on the console 110 such as by appending a text or color indicator that the patient's physiology at that location on the artery is such that the patient is not at an increased risk to experience a Silent Clinical Event, the communication of the parameter values themselves separately or as part of one of the previous messages or by any other means well within the skill of one skilled in the art to communicate such a determination.

Although various embodiments of the invention have been described as being executed in the operation of an algorithm by a microprocessor in the COPD 100, the algorithms could be implemented through the use of a logic or neural network. Alternately the algorithms could be established on discrete analog or digital components as part of the COPD 100.

The present invention has been described in connection with certain embodiments, combinations, configurations and relative dimensions. It is to be understood, however, that the description given herein has been given for the purpose of explaining and illustrating the invention and are not intended to limit the scope of the invention. In addition, it is clear than an almost infinite number of minor variations to the form and function of the disclosed invention could be made and also still be within the scope of the invention. Consequently, it is not intended that the invention be limited to the specific embodiments and variants of the invention disclosed. It is to be further understood that changes and modifications to the descriptions given herein will occur to those skilled in the art. Therefore, the scope of the invention should be limited only by the scope of the claims.

## Claims

1. A device for determining whether a patient's physiology puts him or her at an increased risk of experiencing a Silent Clinical Event, comprising:
a tissue - characterization system (10) including an intravascular ultrasound - (IVUS) console (110) electrically connectable to an intravascular ultrasound - (IVUS) catheter (120) configured to acquire RF backscattered data of an artery of interest, in which the intravascular ultrasound-catheter (120) is to be inserted, and a computing device (130) comprising a database (134) and a characterization application (132) electrically connected to the database (134) and adapted to receive IVUS data from the IVUS console (110) or directly from a transducer (122) attached to the end of the catheter (120);
wherein the tissue - characterizing system (10) is configured for ascertaining for one of more locations along the artery of interest the Plaque Burden of the artery, the Minimum Lumen Area of the artery and whether multiple Virtual Histology-Thin-cap Fibroatheromas (VH-TCFA) are present,
a clinical outcome predictor device (100) being operable on the computing device, or being operable separately and operatively connectable to the intravascular ultrasound - console (110), the computing device (130), the characterization application (132) or database (134) or any combination of these,
wherein the clinical outcome predictor device (100) is configured to
determine whether there is a concurrence for the Plaque Burden, the Minimum Lumen Area, and multiple Virtual Histology-Thin-cap Fibroatheromas (VH-TCFA), and the Plaque Burden being between about 30% and about 70%, the Minimum Lumen Area being less than or equal to about 4 mm² and multiple Virtual Histology-Thin-cap Fibroatheromas (VH-TCFAs) being present; and,
communicate whether such concurrence has occurred by indicating the patient's physiological condition has an increased risk of a Silent Clinical Event.

## Patentansprüche

1. Vorrichtung zum Bestimmen, ob die Physiologie eines Patienten ihn oder sie einem erhöhten Risiko aussetzt, ein stilles klinisches Ereignis zu erleiden, umfassend:
ein Gewebe-Charakterisierungssystem (10) enthaltend eine intravaskuläre Ultraschall- (IVUS-) Konsole (110), die elektrisch mit einem intravaskulären Ultraschall-(IVUS-) Katheter (120) verbindbar ist, der konfiguriert ist, um HF-rückgestreute Daten einer Arterie von Interesse zu erfassen, in die der intravaskuläre Ultraschallkatheter (120) eingeführt werden soll, und eine Rechenvorrichtung (130), die eine Datenbank (134) und eine Charakterisierungsanwendung (132) umfasst, die elektrisch mit der Datenbank (134) verbunden und angepasst ist, um von IVUS-Daten von der IVUS-Konsole (110) oder direkt von einem am Ende des Katheters (120) angebrachten Wandler (122) zu empfangen;
wobei das Gewebe charakterisierende System (10) konfiguriert ist, um für eine von mehreren Stellen entlang der Arterie von Interesse die Plaque-Belastung der Arterie, die Mindest-Lumenfläche der Arterie zu bestimmen und ob mehrere virtuellhistologische Fibroatherome mit dünner Kappe (VH-TCFA) vorhanden sind,
wobei eine klinische Ergebnisvorhersagevorrichtung (100) auf der Computervorrichtung betreibbar ist oder separat und funktionsfähig mit der intravaskulären Ultraschallkonsole (110), der Computervorrichtung (130), der Charakterisierungsanwendung (132) oder der Datenbank (134) oder einer beliebigen Kombination davon verbunden werden kann,
wobei die klinische Ergebnisvorhersagevorrichtung (100) konfiguriert ist, um zu bestimmen, ob ein Zusammentreffen der Plaque-Belastung, der Mindest-Lumenfläche und mehrerer virtuellhistologischer Fibroatherome mit dünner Kappe (VH-TCFA) vorliegt, und wobei die Plaque-Belastung zwischen etwa 30 % und etwa 70 % liegt, wobei die Mindest-Lumenfläche kleiner oder gleich etwa 4 mm² ist und mehrere virtuellhistologische Fibroatherome mit dünner Kappe (VH-TCFAs) vorhanden sind; und
um mitzuteilen, ob ein solches Zusammentreffen eingetreten ist, indem sie angibt, dass der physiologische Zustand des Patienten ein erhöhtes Risiko für ein stille klinischen Ereignis aufweist.

## Revendications

1. Dispositif pour déterminer si une physiologie d'un patient le met en danger accru d'éprouver un événement clinique silencieux,
comprenant :
un système de caractérisation de tissu (10) incluant une console (110) à ultrasons intravasculaires (IVUS) pouvant être électriquement reliée à un cathéter (120) à ultrasons intravasculaires (IVUS) configuré pour acquérir des données rétrodiffusées RF d'une artère d'intérêt, dans lequel le cathéter à ultrasons intravasculaire (120) doit être inséré, et un dispositif de calcul (130) comprenant une base de données (134) et une application de caractérisation (132) électriquement reliée à la base de données (134) et conçue pour recevoir des données IVUS en provenance de la console IVUS (110) ou directement en provenance d'un transducteur (122) attaché à l'extrémité du cathéter (120) ;
dans lequel le système de caractérisation de tissu (10) est configuré pour vérifier, pour l'un de plusieurs emplacements le long de l'artère d'intérêt, le dépôt de plaque sur les parois de l'artère, la surface minimale de lumière de l'artère et si de multiples fibro-athéromes à coiffe fine à histologie virtuelle (VH-TCFA) sont présents,
un dispositif prédicteur de résultat clinique (100) pouvant être mis en œuvre sur le dispositif de calcul, ou pouvant être mis en œuvre séparément et pouvant être relié de manière opérationnelle à la console (110) à ultrasons intravasculaires, au dispositif de calcul (130), à l'application de caractérisation (132) ou à une base de données (134) ou n'importe quelle combinaison de ceux-ci,
dans lequel le dispositif prédicteur de résultat clinique (100) est configuré pour déterminer s'il y a une concurrence pour le dépôt de plaque, la surface minimale de lumière et de multiples fibro-athéromes à coiffe fine à histologie virtuelle (VH-TCFA), et le dépôt de plaque étant entre environ 30 % et environ 70 %, la surface minimale de lumière étant inférieure ou égale à environ 4 mm² et de multiples fibro-athéromes à coiffe fine à histologie virtuelle (VH-TCFA) étant présents ; et
communiquer si une telle concurrence s'est produite en indiquant que l'état physiologique du patient présente un risque accru d'un événement clinique silencieux.
